# EUROPEAN PATENT APPLICATION

(11) **EP 4 821 919 A1**
(43) Date of publication of application: **14.10.2026**
(21) Application number: 25738301.8
(22) Date of filing: 02.01.2025
(51) Int. Cl.: C07D 233/90, C07D 249/10, C07H 19/067, C12N 15/113, A61K 31/713, A61K 48/00

(54) **MODIFIED NUCLEOTIDE MONOMER AND USE THEREOF**

(30) Priority: 08.01.2024 CN 202410024142
(71) Applicant: Hitgen Inc., Chengdu, Sichuan 610200 (CN)
(72) Inventor: LI, Jin, Chengdu, Sichuan 610200 (CN); CHEN, Liu, Chengdu, Sichuan 610200 (CN); HU, Yongjian, Chengdu, Sichuan 610200 (CN); LUO, Huadong, Chengdu, Sichuan 610200 (CN); MA, Huiyong, Chengdu, Sichuan 610200 (CN); LIU, Guansai, Chengdu, Sichuan 610200 (CN)
(74) Representative: Vitina, Maruta
(86) International application number: PCT/CN2025/070080
(87) International publication number: WO 2025/148770

(57) **Abstract**

A nucleotide monomer represented by formula (I), or a stereoisomer, a deuterated compound, a fluorinated compound, or a pharmaceutically acceptable salt thereof: 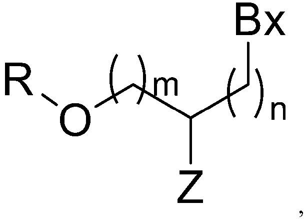 in which Bx is 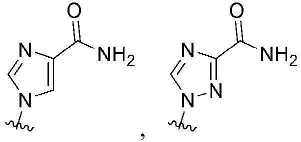 or 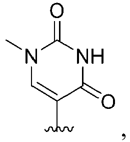 R is hydrogen or a hydroxyl protecting group, m is 1, 2 or 3, n is 1, 2 or 3, and Z is a hydroxyl group, a phosphate ester group, a phosphorothioate group or a phosphoramidite group. A use of the nucleotide monomer as an intermediate in the preparation of small interfering ribonucleic acid (siRNA) is also provided. An siRNA, a pharmaceutical composition including the siRNA and a pharmaceutically acceptable carrier, and an siRNA conjugate formed by conjugating the above siRNA to a conjugate molecule are also provided.

## Description

### TECHNICAL FIELD

This application relates to biochemistry, and more particularly to a modified nucleotide monomer and an application thereof in a small interfering ribonucleic acid (siRNA) molecule.

### BACKGROUND

In recent years, considerable progress has been achieved in the development of small interfering ribonucleic acid (siRNA) druggability. As pharmacologically active ingredients, siRNAs are well known in the art, and the mechanism of RNA interference (RNAi) mediated by siRNAs is characterized by an endonuclease complex. At the genetic level, siRNA can potentially treat almost all diseases, including tumors, infectious diseases and genetic disorders, and thus siRNA has become one of the most popular research areas in biomedicine. Theoretically, siRNA therapeutics can target any target gene in pathogenic genomes, and can provide potent and long-lasting therapeutic effects, significantly reduce administration frequency, and improve patient compliance, thereby achieving better therapeutic outcomes.

It has been demonstrated that one major factor contributing to RNAi off-target effects is that certain fragments within the siRNA sequence that bind to a target messenger RNA (mRNA) may function similarly to microRNAs (miRNAs). These fragments can bind to the 3'-untranslated region (3'-UTRs) of other mRNAs and suppress mRNA translation and stability. miRNAs primarily recognize target genes through base pairing between their seed region (positions 2-8 at the 5'-end) and the target mRNA. It has also been demonstrated that off-target effects induced by siRNA mainly arise from base complementarity between the seed region of the siRNA antisense strand and one or more mRNAs. Therefore, the sequence of the seed region of the antisense strand may affect the expression of multiple genes, and is a primary factor in the side effects of RNAi therapeutics.

In order to effectively address off-target effects in the seed region of the antisense strand, Alnylam modified nucleoside monomers located within the seed region and developed a novel class of nucleoside monomers, i.e., glycerol nucleic acid (GNA). Incorporation of such GNA into the seed region of the antisense strand of the siRNA was found to reduce RNAi off-target effects and enhance drug safety. Thus, off-target effects in the seed region of the antisense strand can be reasonably addressed through modification of the nucleotide monomers within the seed region.

The nucleotide monomer of the present disclosure is introduced at specific positions within the seed region of the antisense strand of the siRNA. Such modification of the siRNA sequence significantly reduces off-target effects of siRNA without impairing target gene silencing activity of siRNA, thereby expanding the safety window of the siRNA drug molecules. Therefore, the present disclosure has great value for the development and clinical application of siRNA therapeutics.

### SUMMARY

An object of the disclosure is to provide a nucleotide monomer and an application thereof. In order to achieve the above object, the following technical solutions are adopted.

In a first aspect, this application provides a nucleotide monomer represented by formula (I), or a stereoisomer, a deuterated compound, a fluorinated compound, or a pharmaceutically acceptable salt thereof: 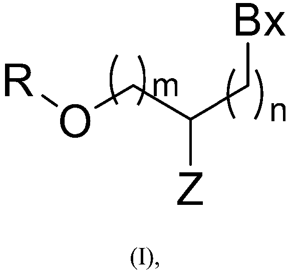
wherein Bx is selected from the group consisting of 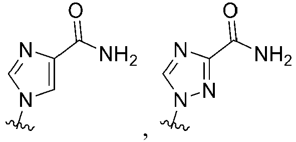 and 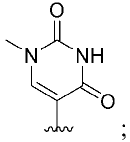
R is hydrogen or a hydroxyl protecting group;
m is 1, 2 or 3, preferably, m is 1;
n is 1, 2 or 3, preferably, n is 1; and
Z is selected from the group consisting of a hydroxyl group, a phosphate ester group, a phosphorothioate group and a phosphoramidite group.

In some embodiments, the nucleotide monomer is represented by formula (II): 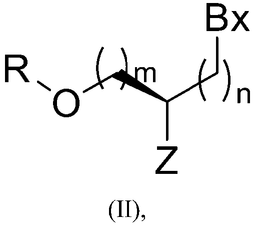
wherein Bx is selected from the group consisting of 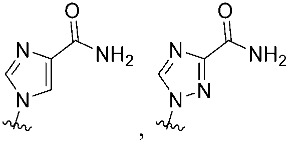 and 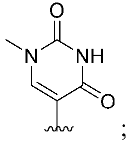
R is hydrogen or a hydroxyl protecting group;
m is 1, 2 or 3, preferably, m is 1;
n is 1, 2 or 3, preferably, n is 1; and
Z is selected from the group consisting of a hydroxyl group, a phosphate ester group, a phosphorothioate group and a phosphoramidite group.

In some embodiments, R is selected from the group consisting of hydrogen, 4,4'-dimethoxytrityl and 4-methoxytrityl; and
Z is a hydroxyl group or

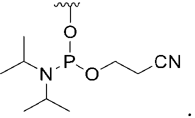

In some embodiments, the nucleotide monomer is selected from the group consisting of 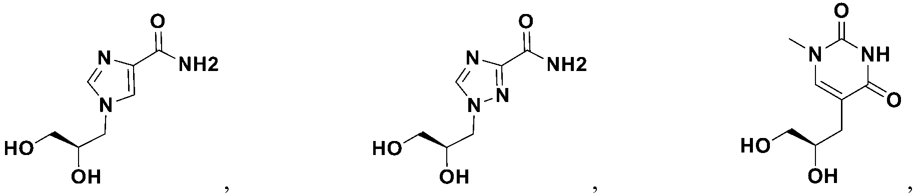 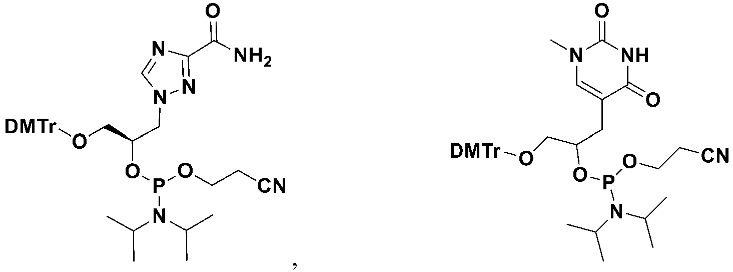 and

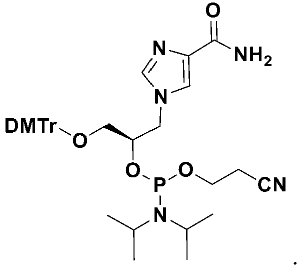

In a second aspect, this application provides a use of the above nucleotide monomer as an intermediate in preparation of oligonucleotide. Preferably, the oligonucleotide is small interfering ribonucleic acid (siRNA).

Preferably, the present application provides a use of the above nucleotide monomer as an intermediate for preparing a seed region of an antisense strand of the siRNA. Specifically, the seed region corresponds to nucleotides 2 to 8 starting from the 5'-end of the antisense strand of the siRNA.

In a third aspect, this application provides an siRNA, comprising:
a sense strand; and
an antisense strand;
wherein the sense strand and the antisense strand each comprise 15 to 45 modified or unmodified nucleotides;
the sense strand and the antisense strand are partially complementary to each other to form a double-stranded region; and
   a seed region of the antisense strand comprises at least one nucleotide having a structure represented by formula (V), and the at least one nucleotide is covalently linked to the rest of the siRNA at a position of 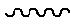 : 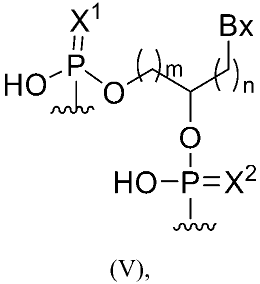
   wherein Bx is selected from the group consisting of 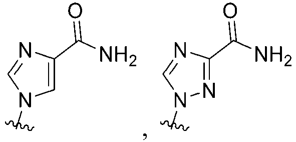 and 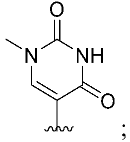
   m is 1, 2 or 3, preferably, m is 1;
   n is 1, 2 or 3, preferably, n is 1; and
   X¹ and X² are each independently O or S.

In some embodiments, the at least one nucleotide is represented by formula (VI): 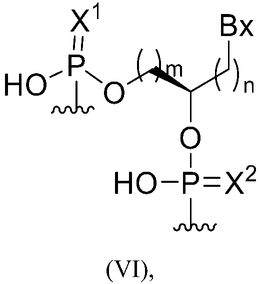
wherein Bx is selected from the group consisting of 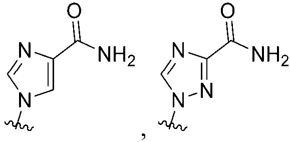 and 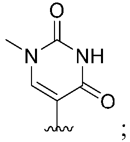
m is 1, 2 or 3, preferably, m is 1;
n is 1, 2 or 3, preferably, n is 1; and
X¹ and X² are each independently O or S.

In some embodiments, the at least one nucleotide is selected from the group consisting of

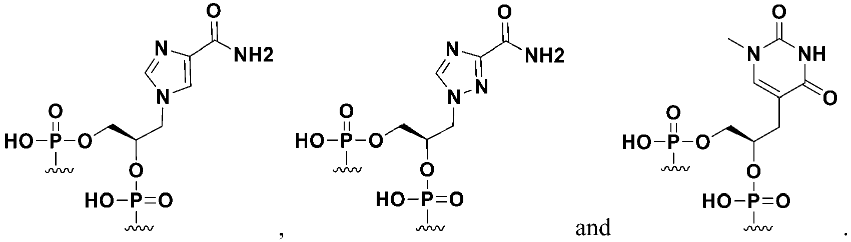

In some embodiments, at least one of nucleotides in the sense strand and/or the antisense strand has the structure represented by formula (V).

In some embodiments, in the 5' to 3' direction, at least one of nucleotides at positions 5, 6, 7, and 8 of the seed region of the antisense strand has the structure represented by formula (V).

In some embodiments, in the 5' to 3' direction, at least one of nucleotides at the positions 5, 6 and 7 of the seed region of the antisense strand has the structure represented by formula (V).

In some embodiments, in the 5' to 3' direction, a nucleotide at position 5 of the seed region of the antisense strand has the structure represented by formula (V).

In some embodiments, in the 5' to 3' direction, a nucleotide at position 6 of the seed region of the antisense strand has the structure represented by formula (V).

In some embodiments, in the 5' to 3' direction, a nucleotide at position 7 of the seed region of the antisense strand has the structure represented by formula (V).

In some embodiments, in the 5' to 3' direction, a nucleotide at position 8 of the seed region of the antisense strand has the structure represented by formula (V).

In some embodiments, in the 5' to 3' direction, a nucleotide at position 5 of the seed region of the antisense strand has the structure represented by formula (V), and nucleotides at positions 2, 6, 14 and 16 of the antisense strand are each independently a 2'-fluoro-modified nucleotide.

In some embodiments, in the 5' to 3' direction, a nucleotide at position 6 of the seed region of the antisense strand has the structure represented by formula (V), and nucleotides at positions 2, 14 and 16 of the antisense strand are each independently a 2'-fluoro-modified nucleotide.

In some embodiments, in the 5' to 3' direction, a nucleotide at position 7 of the seed region of the antisense strand has the structure represented by formula (V), and nucleotides at positions 2, 6, 14 and 16 of the antisense strand are each independently a 2'-fluoro-modified nucleotide.

In some embodiments, in the 5' to 3' direction, a nucleotide at position 8 of the seed region of the antisense strand has the structure represented by formula (V), and nucleotides at positions 2, 6, 14 and 16 of the antisense strand are each independently a 2'-fluoro-modified nucleotide.

In some embodiments, nucleotides at the remaining positions of the antisense strand are each independently a 2'-methoxy-modified nucleotide.

In some embodiments, at least one phosphodiester group in the sense strand and/or the antisense strand is modified by a modifying group. The modifying group is configured such

that the siRNA exhibits increased stability in a biological sample or environment. In some embodiments, the at least one phosphodiester group is a phosphorothioate diester group.

In some embodiments, the at least one phosphodiester group is present at at least one position selected from the group consisting of:
between the first nucleotide and the second nucleotide at the 5'-end of the sense strand,
between the second nucleotide and the third nucleotide at the 5'-end of the sense strand,
between the first nucleotide and the second nucleotide at the 5'-end of the antisense strand,
between the second nucleotide and the third nucleotide at the 5'-end of the antisense strand, and
between the twenty-first nucleotide and the twenty-second nucleotide at the 5'-end of the antisense strand.

In some embodiments, a plurality of phosphodiester groups in the sense strand and/or the antisense strand are each modified by a modifying group.

In some embodiments, a plurality of phosphodiester groups in the sense strand and/or the antisense strand are each modified by a modifying group, wherein the plurality of phosphodiester groups are each independently a phosphorothioate diester group present at positions selected from the group consisting of:
between the first nucleotide and the second nucleotide at the 5'-end of the sense strand,
between the second nucleotide and the third nucleotide at the 5'-end of the sense strand,
between the first nucleotide and the second nucleotide at the 5'-end of the antisense strand,
between the second nucleotide and the third nucleotide at the 5'-end of the antisense strand, and
between the twenty-first nucleotide and the twenty-second nucleotide at the 5'-end of the antisense strand.

In some embodiments, the sense strand comprises the nucleotide sequence (5' to 3' direction) represented by:
mN*mN*mNmNmNmN/fN/mN/fN/fN/fN/mNmNmNmNmNmNmNmNmNmN (SEQ ID NO: 17).

In some embodiments, the antisense strand comprises the nucleotide sequence (5' to 3' direction) represented by:
mN*/fN/*mNmN/V/fN/mNmNmNmNmNmNmN/fN/mN/fN/mNmNmNmNmN*mN *Mn (SEQ ID NO: 18), mN*/fN/*mNmNmN/V/mNmNmNmNmNmNmN/fN/mN/fN/mNmNmNmNmN*mN*mN (SEQ ID NO: 19), or mN*/fN/*mNmNmN/fN/V/mNmNmNmNmNmN/fN/mN/fN/mNmNmNmNmN*mN*mN (SEQ ID NO:20).

In the above sense and antisense strands, "mN" represents any 2'-methoxy-modified nucleoside, where the nucleobase of the 2'-methoxy-modified nucleoside is selected from C, G, U, A and T; "fN" represents any 2'-fluoro-modified nucleoside, where the nucleobase of the 2'-fluoro-modified nucleoside is selected from C, G, U, A and T; "V" represents the nucleotide having the structure represented by formula (V) of the present disclosure; and "*" indicates that the linkage between two nucleotides adjacent to the left and right of the symbol "*" is a phosphorothioate linkage.

In a fourth aspect, this application provides a pharmaceutical composition, comprising the above siRNA and a pharmaceutically acceptable carrier.

In a fifth aspect, this application provides an siRNA conjugate, which is formed by conjugating the above siRNA to a conjugate molecule.

In a sixth aspect, this application provides a use of the above siRNA, the above pharmaceutical composition, the above siRNA conjugate or a combination thereof in preparation of a drug.

In a seventh aspect, this application provides a method for inhibiting an expression level of a target gene in a cell, comprising:
contacting the cell with an effective amount of the siRNA, the pharmaceutical composition, the siRNA conjugate or a combination thereof of the present disclosure.

The modified nucleotide of the present disclosure can improve the selectivity of the RNA interference (RNAi) drug molecule in target gene silencing and reduce the off-target effect.

After introducing the nucleotide monomer of the disclosure into the seed region of the antisense strand of the siRNA, the potential off-target effect in the seed region of the antisense strand can be significantly reduced, while maintaining or enhancing the siRNA activity, thereby achieving higher selective silencing of the target gene messenger RNA (mRNA). This facilitates expanding the safety window of the siRNA drug, and improving the druggability of the siRNA, thus enabling more effective treatment of diseases caused by the target gene mRNA.

The modified nucleotide of the disclosure can improve the druggability of siRNA drug molecules. In some embodiments, the modified nucleotide of the disclosure can enhance the effect of siRNA in degrading target genes. Therefore, such modified nucleotide can strengthen the pharmacological efficacy exerted by siRNA-mediated gene silencing.

The siRNA conjugate provided by the present disclosure is formed by conjugating an siRNA with a conjugating molecule, and comprises the siRNA of the present disclosure and a conjugating group linked to the siRNA, wherein the conjugating group comprises a linker and a pharmaceutically acceptable targeting group and/or a pharmaceutically acceptable delivery-assisting group. Moreover, the siRNA, the linker, and the targeting group or the delivery-assisting group are covalently or non-covalently linked in sequence. Each targeting group is selected from ligands capable of binding to a cell surface receptor, and each delivery-assisting group is selected from groups capable of increasing the biocompatibility of the siRNA conjugate in the target organ or tissue for delivery.

In some embodiments, the siRNA drug comprising the modified nucleotides of the present disclosure is double-stranded. For a double-stranded siRNA drug, the lengths of the sense and antisense strands are each independently 15-45 nucleotides. In some embodiments, the double-stranded siRNA drug consists of a sense strand and an antisense strand that are at least partially complementary (at least 70% complementary) to each other. The antisense strand contains a region having a sequence that is perfectly complementary (100% complementary) or at least partially complementary (at least 85% complementary) to a sequence in the target mRNA. The sense strand and the antisense strand can be equal in length or differ in length.

In some embodiments, the sense strand is about 19 nucleotides in length, and the antisense strand is about 21 nucleotides in length. In some embodiments, the sense strand is about 21 nucleotides in length, and the antisense strand is about 23 nucleotides in length. The region of perfect or partial complementarity between the sense and antisense strands is generally 15-25 nucleotides in length, and is located at or near the 5'-end of the antisense strand (e.g., the region is 1, 2, 3 or 4 nucleotides away from the 5'-end of the antisense strand and exhibits imperfect or partial complementarity).

For a double-stranded siRNA drug, if additional nucleotides of the sense strand are present, they may be identical to or different from the corresponding sequences in the target mRNA. If additional nucleotides of the antisense strand are present, they may be complementary or non-complementary to the corresponding additional nucleotides of the sense strand (if such nucleotides of the sense strand are present).

In some embodiments, the sense and antisense strands of the double-stranded siRNA drug comprising the modified nucleotides of the present disclosure have the same number of nucleotides. In some embodiments, the sense and antisense strands of the siRNA drug of the present disclosure contain different numbers of nucleotides.

In some embodiments, the sense and antisense strands each contain 1 to 4 phosphorothioate linkages.

In some embodiments, each nucleotide in the siRNA of the present disclosure is a modified or unmodified nucleotide. In the context of this disclosure, the term "modified nucleotide" refers to a nucleotide or nucleotide analog formed by substituting the hydroxyl group at the 2' position of the ribosyl group of a nucleotide with another group, or a nucleotide in which the nucleobase is modified. The modified nucleotide would not lead to obvious attenuation or loss of the function of the siRNA in regulating gene expression. For example, modified nucleotides disclosed by Jonathan et. al. may be selected for use ( J. K. Watts, G. F. Deleavey, and M. J. Damha, Chemically modified siRNA: tools and applications. Drug Discov. Today, 2008, 13(19-20): 842-55).

In some embodiments, the siRNA drug comprising the modified nucleotides inhibits expression of a target mRNA in a cell, tissue, or *in vivo.* In some embodiments, a therapeutically effective amount of the siRNA drug comprising the modified nucleotides of the present disclosure is administered *in vivo,* thereby inhibiting expression of the target mRNA *in vivo.*

In some embodiments, the siRNA drug is used for treating, preventing, or controlling a clinical manifestation associated with expression of the target mRNA. In some embodiments, a therapeutically or prophylactically effective amount of one or more types of siRNA drugs is administered to a subject in need of such treatment, prevention, or control. The siRNA drug comprising the modified nucleotides of the present disclosure, and the compositions comprising the siRNA drug of the present disclosure, can be delivered to cells, tumors, tissues or subjects by adopting oligonucleotide delivery techniques known in the art. In general, any method known in the art suitable for delivering nucleic acid molecules *(in vitro* or *in vivo)* can be applied to the siRNA drug comprising one or more modified nucleotides of the present disclosure.

In embodiments where the siRNA drug is double-stranded, a targeting ligand or targeting group, a linking group, or a delivery carrier is covalently bonded to the sense strand to form a conjugate. In some embodiments, the targeting ligand, linking group, and/or delivery carrier is directly or indirectly linked to the 3'-end or 5'-end of the sense strand via a linker. In some embodiments, the targeting ligand or targeting group contains two to four terminal N-acetylgalactosamine (GalNAc) moieties linked to the siRNA drug comprising one or more of the modified nucleotides. It has been demonstrated that trivalent GalNAc structures bind to the asialoglycoprotein receptor (ASGPR) with higher affinity than bivalent or monovalent counterparts (Connolly, D. T.; Townsend, R. R.; Kawaguchi, K.; Bell, W. R.; Lee, Y. C. Binding and Endocytosis of Cluster Glycosides by Rabbit Hepatocytes. Evidence for a Short-Circuit Pathway That Does Not Lead to Degradation. J. Biol. Chem. 1982, 257(2), 939-945).

For definitions of terms used in the present application, unless otherwise indicated, the initial definitions provided for any groups or terms herein shall apply to those groups or terms throughout the entire specification. The terms that are not explicitly defined herein shall be construed with meanings that would be understood by a person skilled in the art based on the disclosure and contextual content.

In the present disclosure, unless otherwise specified, capital letters C, G, U, A and T represent the nucleobase composition of nucleotides, covering both modified and unmodified nucleotides ; "m" indicates that the nucleotide immediately adjacent to the right of the "m" is a 2'-methoxy nucleotide; "f" indicates that the nucleotide immediately adjacent to the right of the "f" is a 2'-fluoro nucleotide; "d" indicates that the nucleotide immediately adjacent to the right of the "d" is a 2'-deoxy nucleotide; "gn" indicates that the nucleotide immediately adjacent to the right of the "gn" is a glycol nucleic acid (GNA) nucleotide; the symbol "*" indicates that the linkage between the two nucleotides adjacent to the symbol "*" (or between a nucleotide and a linker-targeting ligand moiety) is a phosphorothioate linkage; "eVP" indicates that the nucleotide immediately adjacent to its right is an (E)-vinylphosphonate-modified nucleotide; and "iab" indicates an inverted abasic residue.

As used herein, "oligonucleotide" refers to a polymer of linked nucleosides, wherein each nucleoside can be independently modified or unmodified, corresponding to an oligonucleotide sequence containing about 10-50 single-stranded nucleotides or double-stranded nucleotide base pairs. In some embodiments, an oligonucleotide has a nucleobase sequence that is at least partially complementary to a core sequence of a target gene expressed intracellularly. In some embodiments, upon delivery into cells expressing the target gene, the oligonucleotide is capable of modulating expression of the corresponding target gene. Target gene expression can be modulated *in vitro* or *in vivo.* The "oligonucleotide" includes, but is not limited to: single-stranded antisense oligonucleotides, siRNA, double-stranded RNA (dsRNA), microRNA (miRNA), short hairpin RNA (shRNA), ribozymes, interfering RNA molecules, and Dicer substrates.

As used herein, "siRNA" refers to an RNA or RNA-like (e.g., chemically modified RNA) oligonucleotide molecule that is capable of reducing or inhibiting translation of mRNA in a sequence-specific manner.

As used herein, "seed region" refers to a specific region ranging from the second nucleobase to the eighth nucleobase starting from the 5'-end of an oligonucleotide sequence.

siRNA can exert biological effects through the RNA interference mechanism (e.g., by interacting with the mammalian mRNA interference pathway machinery (RNA-induced silencing complex, RISC) to induce mRNA degradation), or through any other alternative mechanism or pathway. Although the siRNA drug as used herein are believed to primarily act through the RNA interference mechanism, the siRNA drug is not bound or restricted to any specific action pathway or mechanism. The siRNA drug includes, but is not limited to, single-stranded antisense oligonucleotides, siRNA, dsRNA, miRNA, shRNA and Dicer substrates. The siRNA drug of the present disclosure consists of an oligonucleotide strand that is at least partially complementary to the target mRNA. In some embodiments, the siRNA drug of the present disclosure is double-stranded, and consists of an antisense strand and a sense strand that is at least partially complementary to the antisense strand.

The terms "silencing", "reducing", "inhibiting", "down-regulating", or "knockdown" mean that when a cell, tissue, organ or animal is treated *in vivo* with the siRNA drug molecule of the present disclosure, the expression level of a given gene is decreased or lowered relative to counterpart a cell, tissue, organ or animal without such treatment.

The term "sequence" or "nucleotide sequence" refers to the order or sequence of nucleobases or nucleotides, represented alphabetically using standard nucleotide nomenclature.

The term "complementary", when used to describe the relationship between a first nucleotide sequence (e.g., the sense strand of an siRNA drug or a target mRNA) and a second nucleotide sequence (e.g., a single-stranded antisense oligonucleotide or the antisense strand of a double-stranded siRNA drug), refers to the ability of an oligonucleotide or polynucleotide containing the first nucleotide sequence and an oligonucleotide or polynucleotide containing the second nucleotide sequence to hybridize under certain conditions (mammalian physiological conditions or analogous *in vitro* conditions) to form base pairing and form a duplex or double-helical structure. Complementary sequences encompass Watson-Crick base pairing or non-Watson-Crick base pairing, and include natural or modified nucleotides or nucleotide analogues, with a degree of complementarity sufficient to meet the above hybridization requirement. For example, for purposes of determining identity or complementarity, the monomers a and Af are complementary to U (or T) and are equivalent to A.

The term "sense strand" refers to the strand of an RNA molecule that carries the nucleotide sequence encoding protein amino acid information, also known as the coding strand, sense strand, or positive-sense strand; the other nucleotide sequence complementary thereto is the antisense strand.

The term "antisense strand" refers to a nucleotide sequence that is substantially reverse-complementary or essentially reverse-complementary to a segment of the mRNA expressed by the target gene, wherein the segment has a length identical to that of the antisense strand.

The term "optional" or "optionally" means that the subsequently described event or circumstance may or may not occur, and the definition covers both scenarios where the event/circumstance occurs and where it does not occur. For example, " heterocyclic group optionally substituted with an alkyl group" means that an alkyl substituent may or may not be present, encompassing both alkyl-substituted heterocyclic groups and unsubstituted heterocyclic groups.

The term "substituted" refers to one or more hydrogen atoms in a group, preferably up to 5, more preferably 1 to 3 hydrogen atoms, independently replaced by a corresponding number of substituents. It is self-evident that substituents are only at their chemically possible positions, and a person skilled in the art can determine possible or impossible substitutions using available techniques and experimental conditions (through experimental or theoretical means). For example, amino or hydroxyl groups having free hydrogen may be unstable when bound to carbon atoms with unsaturated (e.g., olefinic) bonds.

The minimum and maximum carbon atom counts in a hydrocarbon group are designated by prefixes. For example, Cₐ-C_{b} indicates any alkyl group containing "a" to "b" carbon atoms. Accordingly, C₁-C₆ refers to a straight-chain or branched-chain alkyl group containing 1 to 6 carbon atoms.

Alkyl refers to a straight-chain or branched-chain hydrocarbon group derived from an alkane molecule, e.g., methyl (-CH₃), ethyl (-CH₂CH₃), methylene (-CH₂-). An alkyl group may also be part of other groups, such as C₁-C₆ alkoxy and C₁-C₆ alkylamino. Non-limiting examples include methyl, ethyl, n-propyl, isopropyl, n-butyl, isobutyl, tert-butyl, sec-butyl, n-pentyl and n-hexyl. Alkyl may be substituted or unsubstituted; when substituted, the substituents may be at any available bonding site, and the substituents are preferably one or more moieties independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl and carboxylate groups.

As used herein, "protecting group" refers to a labile chemical moiety known in the art for preventing a reactive group (e.g., hydroxy, amino, carboxy, and thiol) from undergoing undesired reactions during synthesis. Protecting groups are typically used selectively and/or orthogonally to protect a site during reactions at other reactive sites, and are subsequently removed to liberate the unprotected group for subsequent reaction steps. In some embodiments, a "substituted" group or substituent contains protecting groups.

Representative hydroxy protecting groups useful in the present disclosure are disclosed in Beaucage et al., Tetrahedron, 1992, 48, 2223-2311, and Greene and Wuts, Protective Groups in Organic Synthesis, Chapter 2, 2d ed., John Wiley & Sons, New York, 1991, each of which is incorporated herein by reference in its entirety. In some embodiments, the protecting group is stable under basic conditions but can be removed under acidic conditions. In some embodiments, non-exclusive examples of hydroxyl protecting groups applicable herein include dimethoxytrityl (DMT), monomethoxytrityl, 9-phenylxanthen-9-yl (Pixyl) and 9-(p-methoxyphenyl)xanthen-9-yl (Mox). In some embodiments, non-exclusive examples of hydroxyl protecting groups applicable herein include trityl (Tr), 4-methoxytrityl (MMTr), 4,4'-dimethoxytrityl (DMTr) and 4,4',4"-trimethoxytrityl (TMTr).

The compounds and compositions described herein may have certain atoms (e.g., N, O or S atoms) in protonated or deprotonated states, depending on the environment in which the compound or composition is placed. Therefore, as used herein, the structures of the present disclosure contemplate that certain functional groups, e.g., OH, SH or NH, may be protonated or deprotonated. The disclosure of the present disclosure is intended to cover the above compounds and compositions regardless of their pH-dependent protonation states, as would be readily understood by one of ordinary skill in the art.

The terms "salt" and "acceptable salts" refer to acid and/or base salts formed by the above compounds or their stereoisomers with inorganic and/or organic acids and bases. Such salts also include zwitterionic salts (inner salts), as well as quaternary ammonium salts such as alkylammonium salts. These salts can be obtained directly during the final isolation and purification of the compound. They may also be obtained by mixing the above compound or stereoisomer thereof with an appropriate amount of an acid or base (e.g., in equivalent amounts). These salts may precipitate in solution and be collected by filtration, or recovered after solvent evaporation, or obtained by lyophilization after reaction in an aqueous medium. This application will be further described below with reference to the embodiments, and these embodiments are not intended to limit this application. Any technologies implemented based on the above description of the present disclosure shall fall within the scope of this application.

### BRIEF DESCRIPTION OF THE DRAWINGS

Fig. 1 shows a ¹H NMR spectrum of compound HG_NA02 in accordance with an embodiment of the present disclosure;
Fig. 2 shows a ¹H NMR spectrum of compound HG_NA03a in accordance with an embodiment of the present disclosure;
Fig. 3 shows a ¹H NMR spectrum of compound HG_NA03b in accordance with an embodiment of the present disclosure;
Fig. 4 shows a liquid chromatography-mass spectrometry (LCMS) chromatogram of the compound HG_NA02;
Fig. 5 shows a LCMS chromatogram of the compound HG_NA03a;
Fig. 6 shows a LCMS chromatogram of the compound HG_NA03b;
Fig. 7 shows on-target activity of small interfering ribonucleic acids (siRNAs) as determined in Test Example 1 of the present disclosure; and
Fig. 8 shows off-target activity of the siRNAs as determined in Test Example 1 of the present disclosure.

### DETAILED DESCRIPTION OF EMBODIMENTS

Structures of the compounds were characterized by nuclear magnetic resonance (NMR) and mass spectrometry (MS). NMR chemical shifts (δ) are expressed in parts per million (ppm, 10⁻⁶). NMR measurements were performed using Bruker^{®} Avance III^{®} 400 and Bruker^{®} Avance^{®} 300 NMR spectrometers. Deuterated dimethyl sulfoxide (DMSO-d₆), deuterated chloroform (CDCl₃), and deuterated methanol (CD₃OD) were used as deuterated solvents, with tetramethylsilane (TMS) serving as an internal standard.

Measurements by liquid chromatography-mass spectrometry (LC-MS) were performed using a Shimadzu^{®} LC-MS 2020 equipped with an electrospray ionization (ESI) source. High-performance liquid chromatography (HPLC) analysis was performed using a Shimadzu^{®} high-performance liquid chromatograph (Shimadzu^{®} LC-20A). Medium-pressure liquid chromatography (MPLC) separation was performed using a Gilson^{®} GX-281 reversed-phase preparative chromatograph. Silica gel plates for thin-layer chromatography (TLC) were Yantai Huanghai^{®} HSGF254 silica gel plates or Qingdao-produced GF254 silica gel plates. TLC plates with a thickness of 0.4-0.5 mm were adopted for product separation and purification. For column chromatography, Yantai Huanghai^{®} silica gel with 200-300 mesh was generally used as the stationary phase carrier.

Known starting materials for the present disclosure can be synthesized by adopting or following methods known in the art, or commercially purchased from suppliers including Energy Chemical^{®}, Chengdu Kelong Chemical^{®}, Accela ChemBio^{®}, and J&K Scientific^{®}. Unless otherwise specified, all materials and reagents used in the Examples of the present disclosure are commercially available.

Unless otherwise stated in the Examples, all reactions were carried out under nitrogen atmosphere. Unless otherwise stated in the Examples, the term "solution" refers to aqueous solution. Unless otherwise stated in the Examples, reactions were carried out at room temperature. Unless otherwise stated in the Examples, the unit "M" represents moles per liter (mol/L).

The present application will be described in further detail below in conjunction with the specific embodiments. However, it should be understood that the specific embodiments described herein are merely intended to explain the present disclosure, and are not intended to limit the present disclosure. All technical solutions implemented based on the disclosure of the present disclosure shall fall within the scope of the disclosure defined by the appended claims.

### EXAMPLE 1 Synthesis of (R)-1-[bis(4-methoxyphenyl)(phenyl)methoxy]-3-(3-oxo-1H-1,2,4-triazol-1-yl)propan-2-yl (2-cyanoethyl) diisopropylphosphoramidite

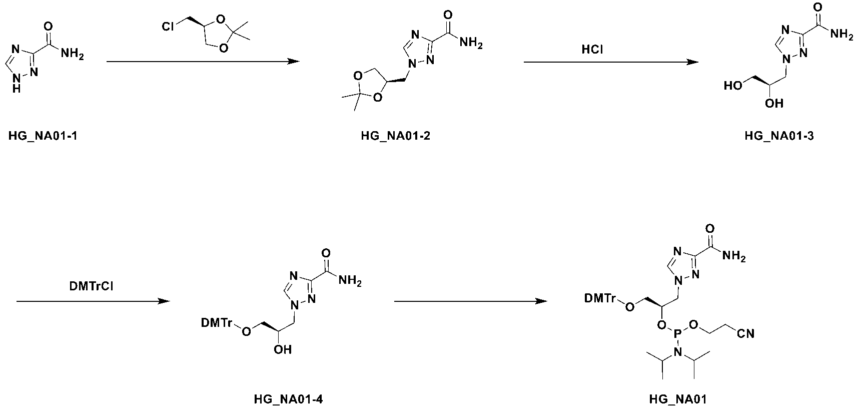

### Step (1) Synthesis of Compound HG_NA01-2

1H-1,2,4-Triazole-3-carboxamide (5.00 g, 44.60 mmol) was added into a 250 mL single-neck round-bottom flask and dissolved with N,N-Dimethylformamide (DMF) (100 mL). Subsequently, (S)-(-)-4-(chloromethyl)-2,2-dimethyl-1,3-dioxolane (8.60 g, 57.10 mmol) and cesium carbonate (29.08 g, 89.21 mmol) were added to obtain a mixture. Then, the mixture was heated to 150°C and stirred for overnight reaction, with LC-MS monitoring. After the rection was completed, the crude reaction solution was directly purified via MPLC to obtain the compound HG_NA01-2 (1.00 g, yield: 10.40%). LCMS (E+) m/z: 227.1 [M+H] ⁺.

### Step (2) Synthesis of Compound HG_NA01-3

The compound HG_NA01-2 (1.00 g, 4.42 mmol) was dissolved with tetrahydrofuran (THF) (30mL) in a 100 mL single-neck round-bottom flask. Concentrated hydrochloric acid (15 mL, 12 M) was added dropwise under stirring. After complete addition, the mixture was heated for reaction overnight, with LC-MS monitoring. After the rection was completed, the reaction solution was concentrated to collect a residue. Anhydrous pyridine (50 mL) was added to the residue followed by concentration to dryness under reduced pressure to obtain the compound HG_NA01-3 (0.89 g, yield: 108.14%). LCMS (E+) m/z: 187.1 [M+H]⁺.

### Step (3) Synthesis of Compound HG_NA01-4

The compound HG_NA01-3 (0.89 g, 4.78 mmol) was dissolved with pyridine (30 mL) in a 100 mL three-neck round-bottom flask, followed by addition of 4,4'-dimethoxytrityl chloride (DMTrCl) (1.62 g, 4.78 mmol). The mixture was stirred at room temperature for reaction for 3 h, with LC-MS monitoring. After the reaction was completed, the solution was concentrated, and the residue was purified by MPLC to obtain the compound HG_NA01-4 (2.20 g, yield: 94.30%). LCMS (E+) m/z: 489.2 [M+H]⁺.

### Step (4) Synthesis of Compound HG_NA01

The compound HG_NA01-4 (1.20 g, 2.46 mmol) was dissolved with anhydrous dichloromethane (DCM) (20 mL) in a 100 mL three-neck round-bottom flask. Then, 2-cyanoethyl N,N-diisopropylchlorophosphoramidite (CEPCl) (0.70 g, 2.94 mmol) and N,N-diisopropylethylamine (DIPEA) (0.63 g, 4.92 mmol) were added. The mixture was stirred at room temperature for reaction for 2 h, with LC-MS monitoring. After the reaction was completed, the crude reaction mixture was directly purified via MPLC to obtain the compound HG_NA01 (0.24 g, yield: 14.19%).

LC-MS (E⁺): m/z = 689.2 [M+H]⁺. ¹H NMR (400 MHz, Chloroform-d) δ 8.27-8.00 (m, 1H), 7.50-7.41 (m, 2H), 7.36-7.18 (m, 8H), 6.97 (s, 1H), 6.83 (t, *J =* 7.8 Hz, 4H), 4.69-4.25 (m, 3H), 3.79 (d, *J =* 2.8 Hz, 6H), 3.56 (dtd, *J* = 47.8, 15.1, 7.1 Hz, 4H), 3.37-3.05 (m, 2H), 2.46 (dt, *J =* 58.1, 6.8 Hz, 2H), 1.17-0.98 (m, 12H).

### EXAMPLE 2 Synthesis of (R)-1-[bis(4-methoxyphenyl)(phenyl)methoxy]-3-(4-oxo-1H-imidazol-1-yl)propan-2-yl (2-cyanoethyl) diisopropylphosphoramidite

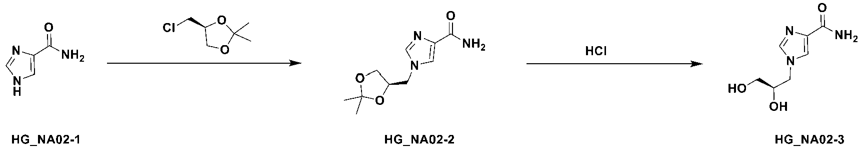

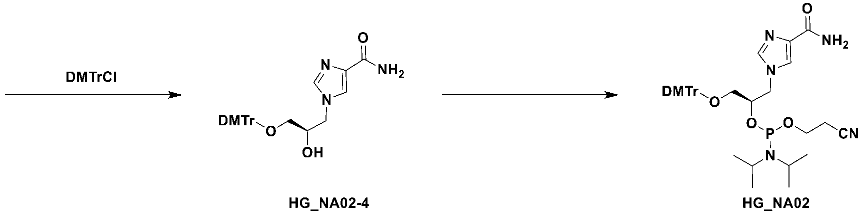

### Step (1) Synthesis of Compound HG_NA02-2

Imidazole-4-carboxamide (4.95 g, 44.60 mmol) was added into a 250 mL single-neck round-bottom flask and dissolved with DMF (100 mL). Subsequently, (S)-(-)-4-(chloromethyl)-2,2-dimethyl-1,3-dioxolane (8.60 g, 57.10 mmol) and cesium carbonate (29.08 g, 89.21 mmol) were added to obtain a mixture. Then, the mixture was heated to 150°C and stirred for overnight reaction, with LC-MS monitoring. After the rection was completed, the crude reaction solution was directly purified via MPLC to obtain the compound HG_NA02-2 (1.40 g, yield: 14.00%). LCMS (E+) m/z: 226.1 [M+H] ⁺.

### Step (2) Synthesis of Compound HG_NA02-3

The compound HG_NA02-2 (1.00 g, 4.42 mmol) was dissolved with THF (30mL) in a 100 mL single-neck round-bottom flask. Concentrated hydrochloric acid (15 mL, 12 M) was added dropwise under stirring. After complete addition, the mixture was heated for reaction overnight, with LC-MS monitoring. After the rection was completed, the reaction solution was concentrated to collect a residue. Anhydrous pyridine (50 mL) was added to the residue followed by concentration to dryness under reduced pressure to obtain the compound HG_NA02-3 (0.87 g, yield: 98.35%). LCMS (E+) m/z: 186.1 [M+H]⁺.

### Step (3) Synthesis of Compound HG_NA02-4

The compound HG_NA02-3 (0.87 g, 4.69 mmol) was dissolved with pyridine (20 mL) in a 100 mL three-neck round-bottom flask, followed by addition of DMTrCl (1.58 g, 4.69 mmol). The mixture was stirred at room temperature for reaction for 3 h, with LC-MS monitoring. After the reaction was completed, the solution was concentrated, and the residue was purified by MPLC to obtain the compound HG_NA02-4 (0.90 g, yield: 39.38%). LCMS (E+) m/z: 488.2 [M+H]⁺.

### Step (4) Synthesis of Compound HG_NA02

The compound HG_NA02-4 (0.55 g, 1.13 mmol) was dissolved with anhydrous DCM (10 mL) in a 100 mL three-neck round-bottom flask. Bis(diisopropylamino)(2-cyanoethoxy)phosphine (0.34 g, 1.13 mmol) and diisopropylammonium tetrazolide (0.10 g, 0.56 mmol) were then added. The mixture was stirred at room temperature for reaction for 1 h, with LC-MS monitoring. After the reaction was completed, the reaction mixture was extracted with saturated aqueous sodium bicarbonate solution and dichloromethane. The organic phase was collected and concentrated, and the crude product was purified by HPLC to obtain the compound HG_NA02 (0.38 g, yield: 48.00%).

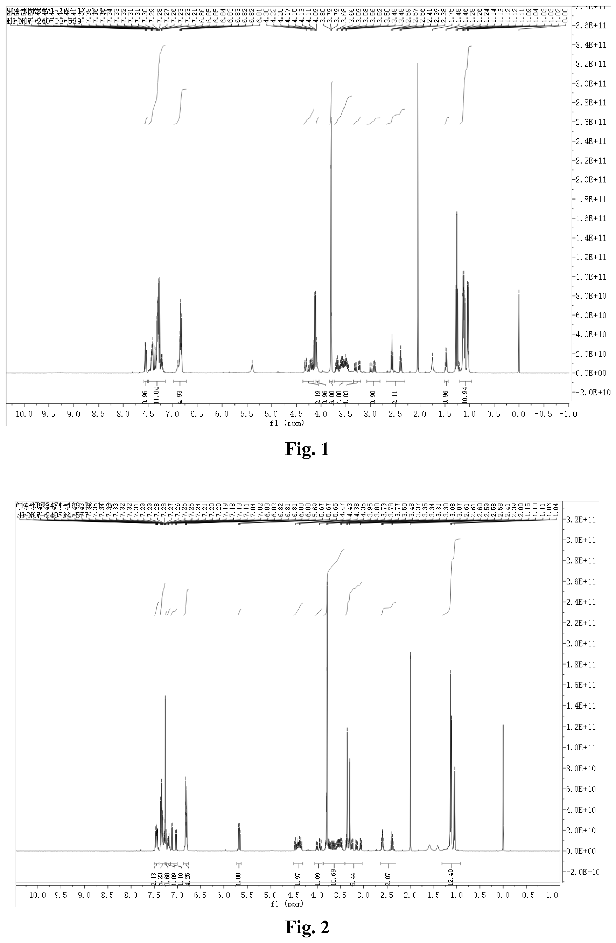

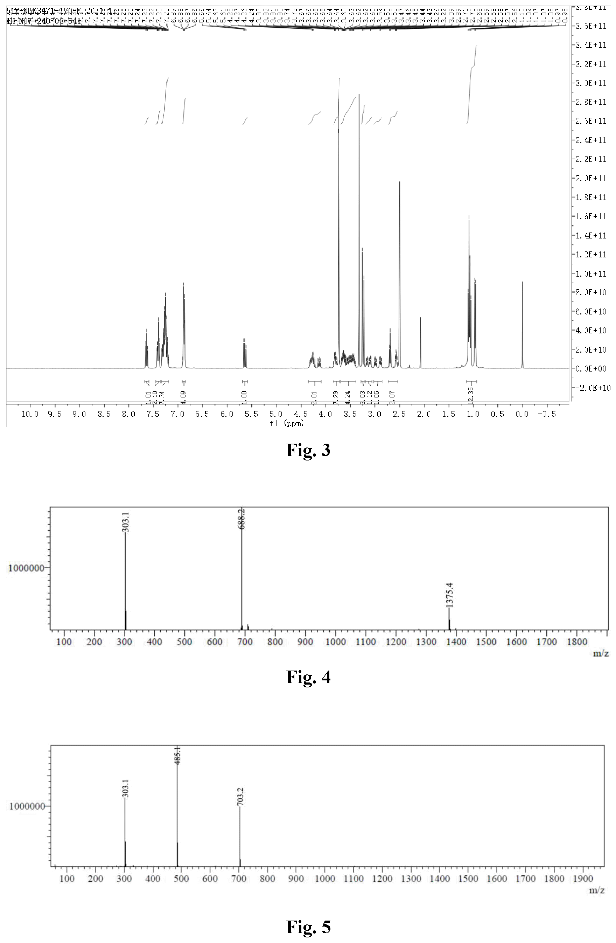

LCMS (E+) m/z: 688.2 [M+H]⁺ (referring to Fig. 4). ¹H NMR (400 MHz, Chloroform-d) δ 7.55 (dd, *J = 7.5,* 1.2 Hz, 1H), 7.51-7.12 (m, 11H), 6.97-6.76 (m, 5H), 4.35-4.04 (m, 3H), 3.80 (d, *J* = 2.7 Hz, 6H), 3.73-3.43 (m, 4H), 3.27 (ddd, *J =* 32.1, 9.8, 4.5 Hz, 1H), 2.95 (ddd, *J* = 28.6, 9.6, 7.3 Hz, 1H), 2.69-2.32 (m, 2H), 1.52-1.44 (m, 1H), 1.19-0.97 (m, 12H) (referring to Fig. 1).

### EXAMPLE 3 Synthesis of 1-[bis(4-methoxyphenyl)(phenyl)methoxy]-3-(1-methyl-2,4-dioxo-1,2,3,4-tetrahydropyridin-5-yl)propan-2-yl (2-cyanoethyl) diisopropylphosphoramidite

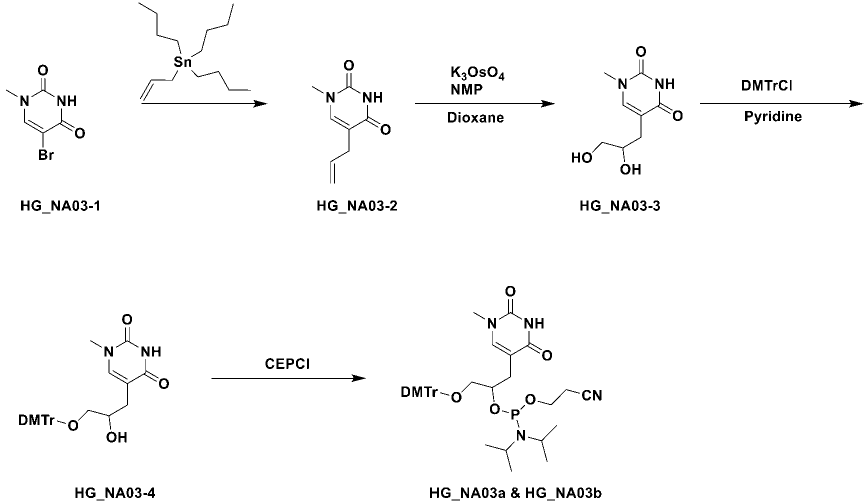

### Step (1) Synthesis of Compound HG_NA03-2

5-Bromo-1-methylpyrimidine-2,4(1H,3H)-dione (6.00 g, 29.27 mmol) was added into a 250 mL reaction flask and dissolved with DMF (100 mL). Subsequently, allyltributylstannane (14.54 g, 43.90 mmol), Pd(PPh₃)₂Cl₂ (2.05 g, 2.93 mmol) and lithium fluoride (0.76 mg, 2.93 mmol) were added to obtain a mixture. Then, the reaction system was purged with nitrogen three times, and the mixture was heated to 110°C and stirred for overnight reaction, with LC-MS monitoring. After the rection was completed, the crude reaction solution was directly purified via MPLC to obtain the compound HG_NA03-2 (1.00 g, yield: 20.56%). LCMS (E+) m/z: 167.1 [M+H] ⁺.

### Step (2) Synthesis of Compound HG_NA03-3

The compound HG_NA03-2 (1.00 g, 6.50 mmol) was dissolved with 1,4-dioxane (10 mL) in a 100 mL single-neck round-bottom flask. N-Methylmorpholine N-oxide (NMO, 0.24 g, 2.07 mmol) and potassium osmate dihydrate (0.60 g, 0.20 mmol) were then added. The mixture was stirred at room temperature for 0.5 h for reaction, with LC-MS monitoring. After the rection was completed, the reaction solution was directly purified via MPLC to obtain the compound HG_NA03-3 (613.00 mg, yield: 50.61%). LCMS (E+) m/z: 201.1 [M+H] ⁺.

### Step (3) Synthesis of Compound HG_NA03-4

The compound HG_NA03-3 (613.00 mg, 3.06 mmol) was dissolved with anhydrous pyridine (20 mL) in a 100 mL single-neck round-bottom flask, followed by addition of DMTrCl (1.04 g, 3.06 mmol). The mixture was stirred at room temperature for reaction for 2 h, with LC-MS monitoring. After the reaction was completed, the solution was concentrated, and the residue was purified by column chromatography to obtain the compound HG_NA03-4 (1.24 g, yield: 80.58%). LCMS (E+) m/z: 503.2 [M+H] ⁺.

### Step (4) Synthesis of Compound HG_NA03a and HG_NA03b

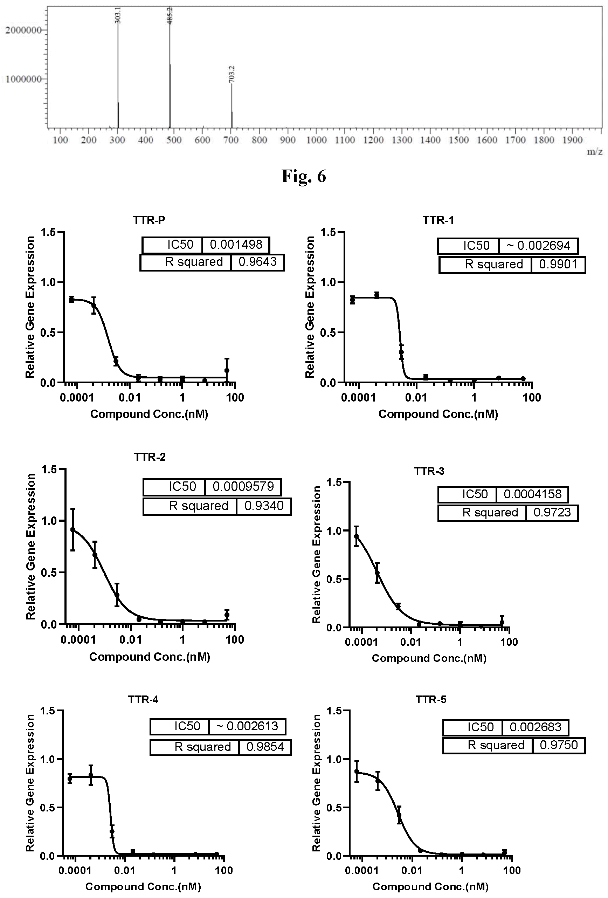

The compound HG_NA03-4 (489.00 mg, 0.97 mmol) was dissolved with DCM (20 mL) in a 100 mL single-neck round-bottom flask. DIPEA (251.51 mg, 1.95 mmol) and CEPCl (253.32 mg, 1.07 mmol) were then added. The mixture was stirred at room temperature for reaction for 2 h, with LC-MS monitoring. After the reaction was completed, the reaction mixture was purified by column chromatography to obtain the compound HG_NA03a and HG_NA03b (150.00 mg each, total yield: 46.65%). LCMS (E+) m/z: 703.2 [M+H] ⁺ (referring to Figs. 5-6).

HG_NA03a: ¹H NMR (400 MHz, Chloroform-d) δ 7.51-7.40 (m, 2H), 7.39-7.23 (m, 6H), 7.22-7.15 (m, 1H), 7.08 (dd, *J=* 34.1, 7.8 Hz, 1H), 6.90-6.72 (m, 4H), 5.67 (dd, *J=* 7.8, 6.0 Hz, 1H), 4.56-4.29 (m, 2H), 3.97 (ddd, *J =* 29.7, 12.3, 3.9 Hz, 1H), 3.86-3.42 (m, 10H), 3.41-3.00 (m, 5H), 2.67-2.29 (m, 2H), 1.33-0.96 (m, 12H) (referring to Fig. 2).

HG_NA03b: ¹H NMR (400 MHz, DMSO-d6) δ 7.64 *(t, J=* 7.5 Hz, 1H), 7.45-7.35 (m, 2H), 7.35-7.16 (m, 7H), 6.88 *(dd, J=* 8.9, 3.1 Hz, 4H), 5.64 *(dd, J=* 12.1, 7.8 Hz, 1H), 4.40-4.07 (m, 2H), 3.86-3.70 (m, 7H), 3.69-3.36 (m, 4H), 3.24 (d, *J =* 13.1 Hz, 3H), 3.13 (ddd, *J =* 25.7, 9.7, 4.8 Hz, 1H), 2.94 (ddd, *J=* 40.0, 9.7, 5.1 Hz, 1H), 2.74-2.53 (m, 2H), 1.31-0.75 (m, 12H) (referring to Fig. 3).

### EXAMPLE 4 Synthesis of siRNA

For the sense and antisense strands of the siRNA duplexes disclosed herein, as well as the sense and antisense strands of modified duplexes, deoxynucleoside controlled pore glass (CPG) was adopted as a solid support. The sense strand was synthesized on the solid support, and the antisense strand was synthesized on universal CPG.

Sequence synthesis was performed at a 0.2 µmol scale using a 48-channel synthesizer. A phosphoramidite monomer with a concentration of 0.05 M was used, and 5-(benzylthio)-1H-tetrazole (BTT) with a concentration of 0.3 M was used as an activator.

Cleavage and deprotection of sequences were performed in a 1.5 mL centrifuge tube. Ammonium hydroxide/methylamine (AMA) was adopted in the first step, and triethylamine trihydrofluoride was used in the second step to remove 2'-protecting groups. Sequences with full 2'-modifications required ammonolysis with aqueous ammonia. The sequences after cleavage and deprotection were precipitated with an acetone/ethanol mixture (80:20, v/v), and then redissolved in RNase-free water. Each sequence was characterized by LC-MS to confirm sequence identity, quantified with a spectrophotometer, and tested for purity by HPLC.

After HPLC purification, lyophilization and quality testing, sodium acetate-mediated alcoholic precipitation was performed for salt exchange, followed by desalting with 3 kDa ultrafiltration tubes. After desalting, the sense and antisense strands were quantified by spectrophotometry, mixed at a molar ratio of 1:1, and annealed to form siRNA duplexes. The siRNA sequences employed in the present disclosure are listed in Table 1.

**Table 1 siRNA sequences employed in the present disclosure**

| Compound Number | | Modified sequence information (5' to 3') |
|---|---|---|
| TTR-P | SS | mA*mA*mCmAmGmU/fG/mU/fU/fC/fU/mUmGmCmUm CmUmAmUmAmA (SEQ ID NO:1) |
| | AS | mU*/fU/*mAmUmA/fG/mAmGmCmAmAmGmA/fA/mC/f A/mCmUmGmUmU*mU*Mu (SEQ ID NO:2) |
| TTR-1 | SS | mA*mA*mCmAmGmU/fG/mU/fU/fC/fU/mUmGmCmUm CmUmAmUmAmA (SEQ ID NO:1) |
| | AS | mU*/fU/*mAmU/GNA(A)/fG/mAmGmCmAmAmGmA/fA/ mC/fA/mCmUmGmUmU*mU*mU (SEQ ID NO:3) |
| TTR-2 | SS | mA*mA*mCmAmGmU/fG/mU/fU/fC/fU/mUmGmCmUm CmUmAmUmAmA (SEQ ID NO:1) |
| | AS | mU*/fU/*mAmU/HG_NA02/fG/mAmGmCmAmAmGmA/f A/mC/fA/mCmUmGmUmU*mU*mU (SEQ ID NO:4) |
| TTR-3 | SS | mA*mA*mCmAmGmU/fG/mU/fU/fC/fU/mUmGmCmUm CmUmAmUmAmA (SEQ ID NO:1) |
| | AS | mU*/fU/*mAmUmA/GNA(G)/mAmGmCmAmAmGmA/fA /mC/fA/mCmUmGmUmU*mU*mU (SEQ ID NO:5) |
| TTR-4 | SS | mA*mA*mCmAmGmU/fG/mU/fU/fC/fU/mUmGmCmUm CmUmAmUmAmA (SEQ ID NO:1) |
| | AS | mU*/fU/*mAmUmA/HG_NA02/mAmGmCmAmAmGmA/f A/mC/fA/mCmUmGmUmU*mU*mU (SEQ ID NO:6) |
| TTR-5 | SS | mA*mA*mCmAmGmU/fG/mU/fU/fC/fU/mUmGmCmUm CmUmAmUmAmA (SEQ ID NO:1) |
| | AS | mU*/fU/*mAmUmA/fG/GNA(A)/mGmCmAmAmGmA/fA/ mC/fA/mCmUmGmUmU*mU*mU (SEQ ID NO:7) |
| TTR-6 | SS | mA*mA*mCmAmGmU/fG/mU/fU/fC/fU/mUmGmCmUm CmUmAmUmAmA (SEQ ID NO:1) |
| | AS | mU*/fU/*mAmUmA/fG/HG_NA02/mGmCmAmAmGmA/f A/mC/fA/mCmUmGmUmU*mU*mU (SEQ ID NO:8) |
| AGT-P | SS | mG*mU*mCmAmUmC/fC/mA/fC/fA/fA/mUmGmAmGm AmGmUmAmCmA (SEQ ID NO:9) |
| | AS | mU*/fG/*mUmAmC/fU/mCmUmCmAmUmUmG/fU/mG/f G/mAmUmGmAmC*mG*mA (SEQ ID NO:10) |
| AGT-1 | SS | mG*mU*mCmAmUmC/fC/mA/fC/fA/fA/mUmGmAmGm AmGmUmAmCmA (SEQ ID NO:9) |
| | AS | mU*/fG/*mUmAmC/GNA(T)/mCmUmCmAmUmUmG/fU/ mG/fG/mAmUmGmAmC*mG*mA (SEQ ID NO:11) |
| AGT-2 | SS | mG*mU*mCmAmUmC/fC/mA/fC/fA/fA/mUmGmAmGm AmGmUmAmCmA (SEQ ID NO:9) |
| | AS | mU*/fG/*mUmAmC/HG_NA03a/mCmUmCmAmUmUmG/ fU/mG/fG/mAmUmGmAmC*mG*mA (SEQ ID NO:12) |
| AGT-3 | SS | mG*mU*mCmAmUmC/fC/mA/fC/fA/fA/mUmGmAmGm AmGmUmAmCmA (SEQ ID NO:9) |
| | AS | mU*/fG/*mUmAmC/HG_NA03b/mCmUmCmAmUmUmG /fU/mG/fG/mAmUmGmAmC*mG*mA (SEQ ID NO:13) |
| AGT-4 | SS | mG*mU*mCmAmUmC/fC/mA/fC/fA/fA/mUmGmAmGm AmGmUmAmCmA (SEQ ID NO:9) |
| | AS | mU*/fG/*mUmAmC/fU/GNA(C)/mUmCmAmUmUmG/fU/ mG/fG/mAmUmGmAmC*mG*mA (SEQ ID NO:14) |
| AGT-5 | SS | mG*mU*mCmAmUmC/fC/mA/fC/fA/fA/mUmGmAmGm AmGmUmAmCmA (SEQ ID NO:9) |
| | AS | mU*/fG/*mUmAmC/fU/HG_NA03a/mUmCmAmUmUmG/ fU/mG/fG/mAmUmGmAmC*mG*mA (SEQ ID NO:15) |
| AGT-6 | SS | mG*mU*mCmAmUmC/fC/mA/fC/fA/fA/mUmGmAmGm AmGmUmAmCmA (SEQ ID NO:9) |
| | AS | mU*/fG/*mUmAmC/fU/HG_NA03b/mUmCmAmUmUmG/ fU/mG/fG/mAmUmGmAmC*mG*mA (SEQ ID NO:16) |

In Table 1, "SS" refers to the sense strand, and "AS" refers to the antisense strand; the letter "m" means the nucleotide adjacent thereto from the right side is a 2'-methoxy-modified nucleotide; the letter "f" means the nucleotide adjacent thereto from the right side is a 2'-fluoro-modified nucleotide; the symbol "*" represents that the linkage between two adjacent nucleotides on its left and right is a phosphorothioate linkage; and GNA denotes (S)-glycol nucleic acid. HG_NA02 represents the nucleoside phosphoramidite monomer HG_NA02 obtained in Example 2 by solid-phase synthesis. HG_NA03a and HG_NA03b respectively represent the nucleoside phosphoramidite monomers HG_NA03a and HG_NA03b obtained in Example 3 by solid-phase synthesis.

### TEST EXAMPLE 1 Detection of Cellular On-Target and Off-Target Activity of siRNA Based on psiCHECK^{™} System

### 1. Construction of on-target and off-target psiCHECK plasmids

A pair of plasmids using psiCHECK^{™}-2 vector were constructed for each siRNA compound. The two plasmids were psiCHECK-cm plasmid and psiCHECK-sm plasmid. The psiCHECK-cm plasmid was inserted with a sequence exhibiting a complete match to the antisense strand sequence of the target siRNA compound. The psiCHECK-sm plasmid was inserted with a sequence only matching the seed region (nucleotides 2 to 8 in the 5' to 3' direction of the antisense strand of the siRNA). The psiCHECK-cm plasmid and psiCHECK-sm plasmid were used to detect the on-target activity and off-target activity of the siRNA compound, respectively.

### 2. Cell culture, transfection and dual-luciferase reporter assay

HEK293T cells were cultured in complete DMEM medium (Gibco, supplemented with 10% by volume of fetal bovine serum (FBS)) at 37°C under 5% (by volume) CO₂ until nearly confluent. Subsequently, cells were digested with trypsin and seeded into 96-well plates.

Reverse transfection of siRNAs and plasmids was carried out simultaneously with HEK293T cell seeding. Multiple concentration gradients were set for each test siRNA compound with 3-4 replicate wells for each concentration. Wells containing only cells and Opti-MEM medium were defined as the background group, and wells transfected with plasmid alone were set as the control group. After dilution of test siRNAs and plasmids, HEK293T cell suspensions were transfected with Lipofectamine^{™} 2000. After incubation for 24 h at 37°C in a 5% CO₂ incubator, Renilla luciferase and Firefly luciferase activities were detected following the recommended protocol of the Dual-Glo^{®} Luciferase Assay System (Vazyme). Data processing for test results was as follows. The calculated value represents the remaining percentage of target mRNA expression: 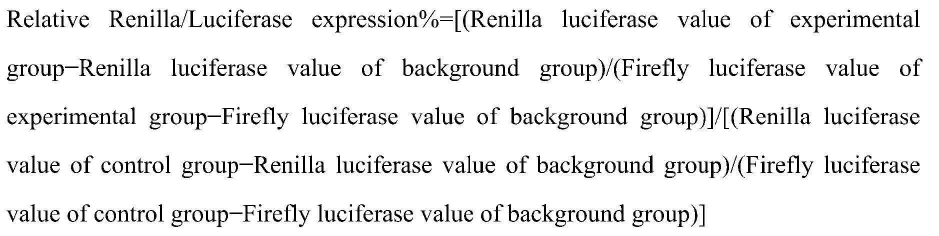 Relative Renilla/Luciferase expression%=[(Renilla luciferase value of experimental group-Renilla luciferase value of background group)/(Firefly luciferase value of experimental group-Firefly luciferase value of background group)]/[(Renilla luciferase value of control group-Renilla luciferase value of background group)/(Firefly luciferase value of control group-Firefly luciferase value of background group)]

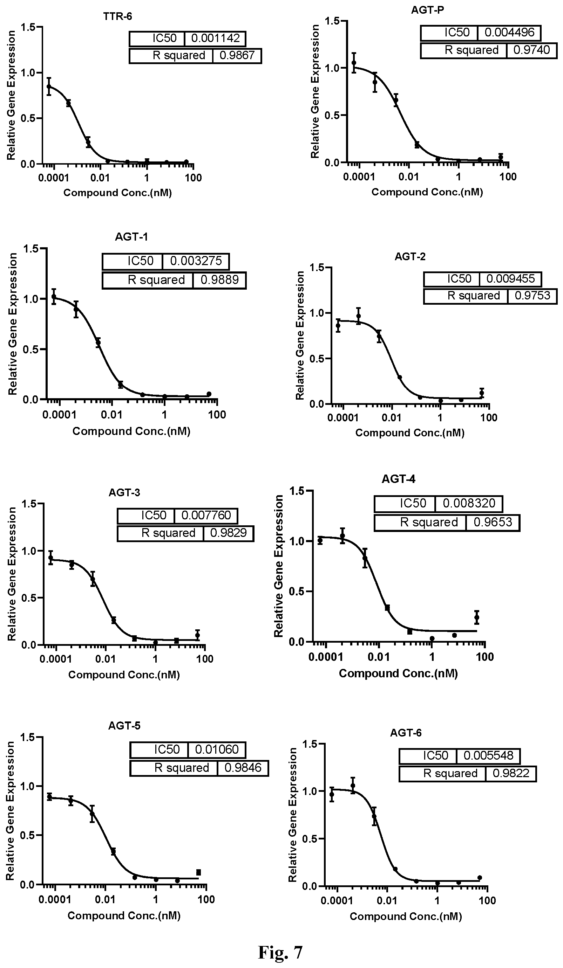

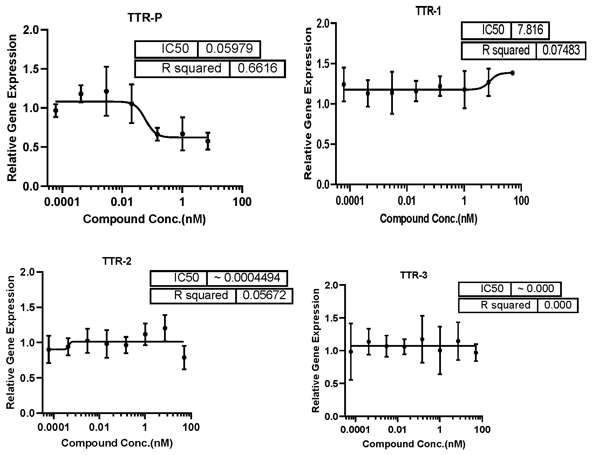

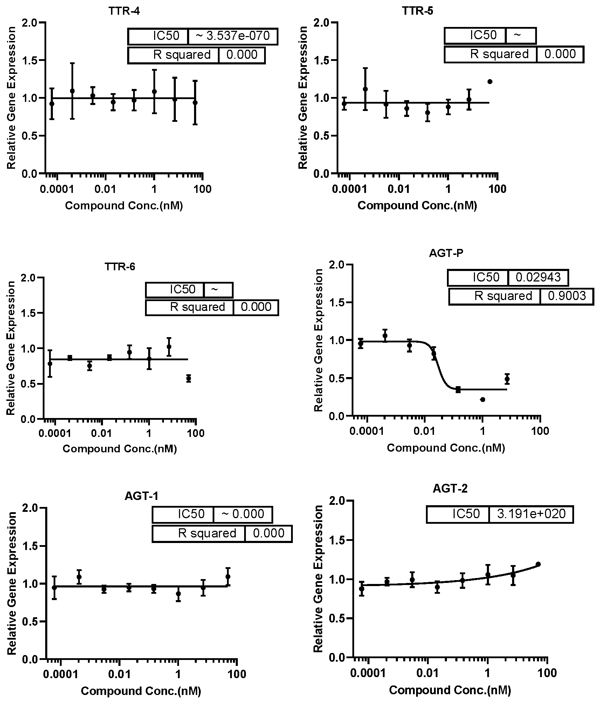

For the on-target activity detection experiment, the HEK293T cell line wase co-transfected with recombinant psiCHECK-cm plasmid and siRNA compounds. The initial concentration of the compounds was set to 50 nM, and 7-fold serial dilution was adopted to prepare eight concentration points: 50 nM, 7.14286 nM, 1.02041 nM, 0.14577 nM, 0.02082 nM, 0.00297 nM, 0.00042 nM, 0.00006 nM. The activity screening results of siRNA compounds are shown in Table 2 and Fig. 7.

**Table2 Test results of psiCHECK^{™}-2 on-target activity**

| Duplex Number | Residual percentage of target mRNA expression (average value) | | | | | | | | *IC*₅₀ value (nM) |
|---|---|---|---|---|---|---|---|---|---|
| | 50 nM | 7.14286 nM | 1.02041 nM | 0.14577 nM | 0.0208 2 nM | 0.0029 7 nM | 0.0004 2 nM | 0.0000 6 nM | |
| TTR-P | 0.1212 | 0.0191 | 0.0370 | 0.0320 | 0.0461 | 0.2121 | 0.7693 | 0.8291 | 0.0015 |
| TTR-1 | 0.0393 | 0.0464 | 0.0258 | 0.0238 | 0.0546 | 0.3037 | 0.8717 | 0.8241 | 0.0027 |
| TTR-2 | 0.0912 | 0.02303 | 0.0274 | 0.0235 | 0.0452 | 0.2825 | 0.6697 | 0.9132 | 0.0010 |
| TTR-3 | 0.0503 | 0.0119 | 0.0285 | 0.0398 | 0.0307 | 0.2158 | 0.5628 | 0.9402 | 0.0004 |
| TTR-4 | 0.0180 | 0.0169 | 0.0073 | 0.0113 | 0.0355 | 0.2529 | 0.8326 | 0.7966 | 0.0026 |
| TTR-5 | 0.0331 | 0.0121 | 0.0192 | 0.0135 | 0.0535 | 0.4225 | 0.7729 | 0.8717 | 0.0027 |
| TTR-6 | 0.0244 | 0.0185 | 0.0260 | 0.0232 | 0.0312 | 0.2382 | 0.6650 | 0.8484 | 0.0011 |
| AGT-P | 0.0555 | 0.0312 | 0.0166 | 0.0348 | 0.1871 | 0.6605 | 0.8499 | 1.0558 | 0.0045 |
| AGT-1 | 0.0531 | 0.0255 | 0.0277 | 0.0447 | 0.1469 | 0.5659 | 0.8938 | 1.0221 | 0.0033 |
| AGT-2 | 0.1212 | 0.0447 | 0.0343 | 0.0714 | 0.2969 | 0.7450 | 0.9665 | 0.8616 | 0.0095 |
| AGT-3 | 0.1019 | 0.0448 | 0.0244 | 0.0669 | 0.2614 | 0.7003 | 0.8504 | 0.9275 | 0.0078 |
| AGT-4 | 0.2407 | 0.0631 | 0.0321 | 0.1047 | 0.3384 | 0.8320 | 1.0546 | 1.0075 | 0.0083 |
| AGT-5 | 0.1246 | 0.0386 | 0.0496 | 0.0704 | 0.3344 | 0.7179 | 0.8503 | 0.8925 | 0.0106 |
| AGT-6 | 0.0893 | 0.0376 | 0.0325 | 0.0514 | 0.1811 | 0.7344 | 1.0567 | 0.9649 | 0.0055 |

For active sequences of transthyretin (TTR)-targeting siRNA, substitution of adenine (A) bases at positions 5 and 7 within the seed region of the antisense strand (AS) with HG_NA02 brought well-preserved on-target activity (Sequence Nos.: TTR-2 and TTR-6). Their activity was even superior to that of modified sequences with GNA(A) substitution (Sequence Nos.: TTR-1 and TTR-5). Substitution of the guanine (G) base at position 6 in the seed region of the antisense strand (AS) with HG_NA02 also retained favorable on-target activity (Sequence No.: TTR-4). These results demonstrate that the monomer HG_NA02 can substitute both A and G bases while maintaining on-target activity. For active sequences of angiotensinogen (AGT)-targeting siRNA, substitution of the thymine (T) base at position 6 within the seed region of the antisense strand (A) with HG_NA03a or HG_NA03b maintained excellent on-target activity (Sequence Nos.: AGT-2 and AGT-3). Substitution of the cytosine (C) base at position 7 in the seed region of the antisense strand (AS) with HG_NA03a or HG_NA03b also maintained excellent on-target activity (Sequence Nos.: AGT-5 and AGT-6), among which AGT-6 showed higher activity than AGT-4 modified with GNA(C). These results indicate that HG_NA03 is able to substitute both T and C bases while maintaining on-target activity.

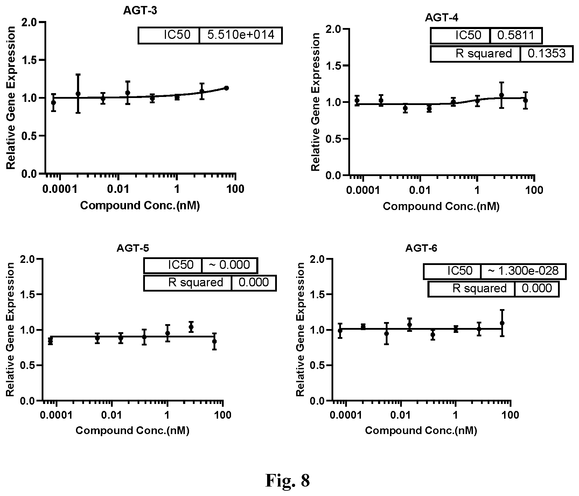

For the off-target activity detection assay, the HEK293T cell line was co-transfected with recombinant psiCHECK-sm plasmid and each siRNA compound. The initial concentration of the test compounds was set to 50 nM, and eight concentration points were prepared by 7-fold serial dilution: 50 nM, 7.14286 nM, 1.02041 nM, 0.14577 nM, 0.02082 nM, 0.00297 nM, 0.00042 nM, 0.00006 nM. The activity screening results of the siRNA compounds are listed in Table 3 and Fig. 8.

**Table 3 Test results of psiCHECK^{™}-2 off-target activity**

| Duplex Number | Residual percentage of target mRNA expression (average value) | | | | | | | | *IC*₅₀ value (nM) |
|---|---|---|---|---|---|---|---|---|---|
| | 50 nM | 7.1428 6 nM | 1.0204 1 nM | 0.1457 7 nM | 0.0208 2 nM | 0.0029 7 nM | 0.0004 2 nM | 0.0000 6 nM | |
| TTR-P | \ | 0.5762 | 0.6698 | 0.6638 | 1.0537 | 1.2141 | 1.1812 | 0.9675 | 0.059 8 |
| TTR-1 | 1.3846 | 1.2672 | 1.1770 | 1.2184 | 1.1597 | 1.1367 | 1.1292 | 1.2400 | >50 |
| TTR-2 | 0.7879 | 1.2044 | 1.1175 | 0.9639 | 0.9814 | 1.0234 | 0.9412 | 0.9000 | >50 |
| TTR-3 | 0.9714 | 1.1453 | 1.0049 | 1.1750 | 1.0605 | 1.0687 | 1.1381 | 0.9850 | >50 |
| TTR-4 | 0.9368 | 0.9809 | 1.0842 | 0.9686 | 0.9449 | 1.0316 | 1.0923 | 0.9225 | >50 |
| TTR-5 | 1.2156 | 0.9787 | 0.8803 | 0.8065 | 0.8600 | 0.9163 | 1.1151 | 0.9233 | >50 |
| TTR-6 | 0.5772 | 1.0213 | 0.8550 | 0.9436 | 0.8709 | 0.7560 | 0.8663 | 0.7867 | >50 |
| AGT-P | \ | 0.4879 | 0.2157 | 0.3482 | 0.8234 | 0.9299 | 1.0575 | 0.9550 | 0.0294 |
| AGT-1 | 1.0928 | 0.9451 | 0.8680 | 0.9327 | 0.9491 | 0.9261 | 1.0886 | 0.9475 | >50 |
| AGT-2 | 1.1924 | 1.0497 | 1.0591 | 0.9838 | 0.8990 | 0.9944 | 0.9699 | 0.8775 | >50 |
| AGT-3 | 1.1292 | 1.0854 | 1.0091 | 0.9931 | 1.0669 | 0.9922 | 1.0537 | 0.9375 | >50 |
| AGT-4 | 1.0233 | 1.0959 | 1.0178 | 1.0034 | 0.9115 | 0.9180 | 1.0227 | 1.0225 | >50 |
| AGT-5 | 0.8351 | 1.0384 | 0.9522 | 0.8985 | 0.8830 | 0.8812 | \ | 0.8375 | >50 |
| AGT-6 | 1.0942 | 1.0110 | 1.0116 | 0.9306 | 1.0720 | 0.9462 | 1.0417 | 0.9875 | >50 |

For active sequences of TTR-targeting siRNA, substitution of the A bases at positions 5 and 7 within the seed region of the antisense strand with HG_NA02 can significantly reduce the off-target activity of the siRNA; substitution of the G base at position 6 in the seed region of the antisense strand with HG_NA02 can also significantly reduce the off-target activity of the siRNA. For active sequences of AGT-targeting siRNA, substitution of the T base at position 6 within the seed region of the antisense strand with HG_NA03a or HG_NA03b can significantly reduce the off-target activity of the siRNA; substitution of the C base at position 7 in the seed region of the antisense strand with HG_NA03a or HG_NA03b can also significantly reduce the off-target activity of the siRNA. These results indicate that siRNAs containing the nucleotide analog monomers disclosed in the present disclosure can effectively reduce off-target activity.

## Claims

1. A nucleotide monomer represented by formula (I), or a stereoisomer, a deuterated compound, a fluorinated compound, or a pharmaceutically acceptable salt thereof: 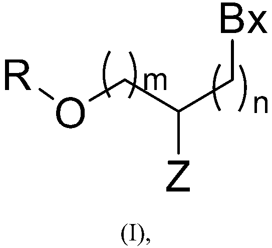
**characterized in that** Bx is selected from the group consisting of 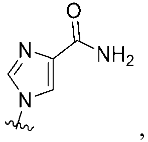 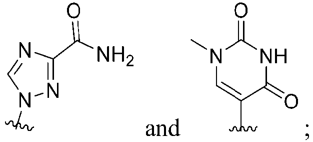
R is hydrogen or a hydroxyl protecting group;
m is 1, 2 or 3; preferably, m is 1;
n is 1, 2 or 3; preferably, n is 1; and
Z is selected from the group consisting of a hydroxyl group, a phosphate ester group, a phosphorothioate group and a phosphoramidite group.

2. The nucleotide monomer according to claim 1, **characterized in that** the nucleotide monomer is represented by formula (II): 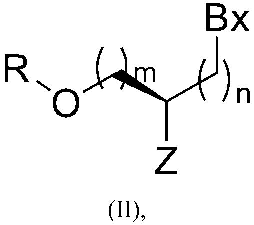
wherein Bx is selected from the group consisting of 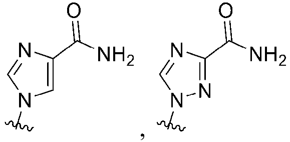 and 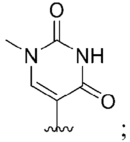
R is hydrogen or a hydroxyl protecting group;
m is 1, 2 or 3; preferably, m is 1;
n is 1, 2 or 3; preferably, n is 1; and
Z is selected from the group consisting of a hydroxyl group, a phosphate ester group, a phosphorothioate group and a phosphoramidite group.

3. The nucleotide monomer according to claim 1 or 2, **characterized in that** R is selected from the group consisting of hydrogen, 4,4'-dimethoxytrityl and 4-methoxytrityl; and Z is a hydroxyl group or

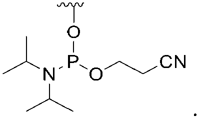

4. The nucleotide monomer according to any one of claims 1-3, **characterized in that** the nucleotide monomer is selected from the group consisting of

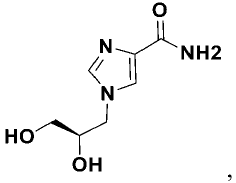

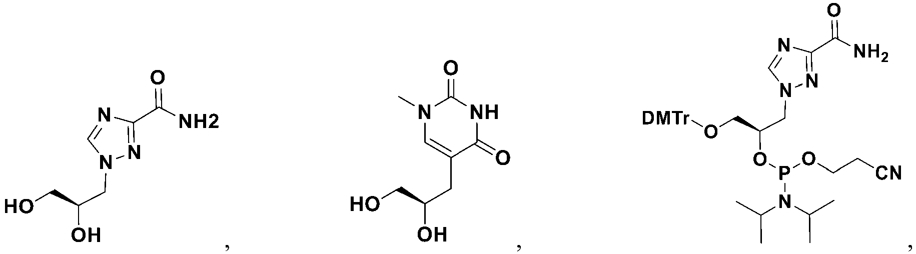

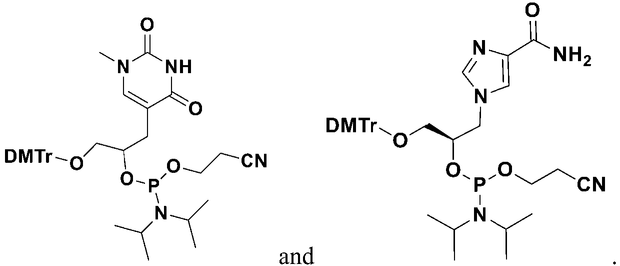

5. The nucleotide monomer according to any one of claims 1-4 for use in the preparation of an oligonucleotide, **characterized in that** the oligonucleotide is a small interfering ribonucleic acid (siRNA).

6. The nucleotide monomer for use according to claim 5, **characterized in that** the nucleotide monomer is used as an intermediate in the preparation of a seed region of an antisense strand of the siRNA.

7. An siRNA, comprising:
a sense strand; and
an antisense strand;
**characterized in that** the sense strand and the antisense strand each comprise 15 to 45 modified or unmodified nucleotides;
the sense strand and the antisense strand are partially complementary to each other to form a double-stranded region; and
a seed region of the antisense strand comprises at least one nucleotide represented by formula (V), and the at least one nucleotide represented by formula (V) is covalently linked to the rest of the siRNA at a site of 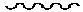 : 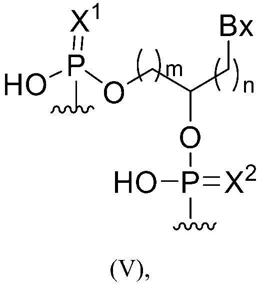
wherein Bx is selected from the group consisting of 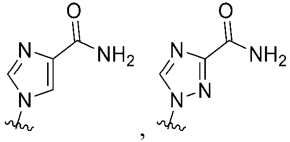 and 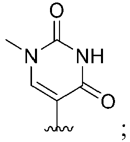
m is 1, 2 or 3; preferably, m is 1;
n is 1, 2 or 3; preferably, n is 1; and
X¹ and X² are each independently O or S.

8. The siRNA according to claim 7, **characterized in that** the at least one nucleotide represented by formula (V) is represented by formula (VI): 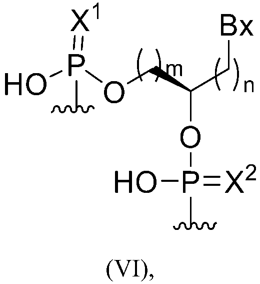
wherein Bx is selected from the group consisting of 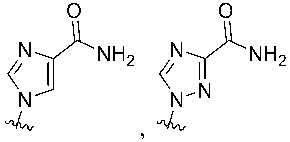 and 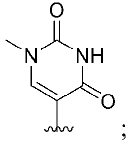
m is 1, 2 or 3; preferably, m is 1;
n is 1, 2 or 3; preferably, n is 1; and
X¹ and X² are each independently O or S.

9. The siRNA according to claim 7 or 8, **characterized in that** the at least one nucleotide represented by formula (V) is selected from the group consisting of

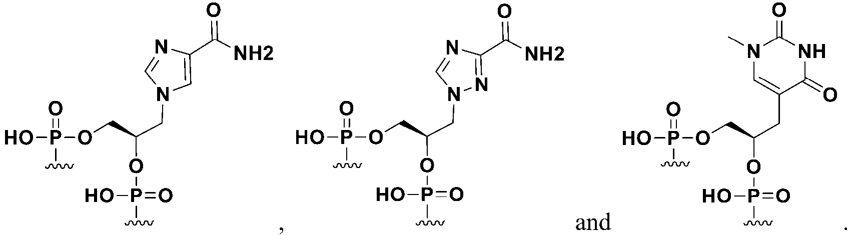

10. The siRNA according to claim 7 or 8, **characterized in that** in the 5' to 3' direction, at least one of nucleotides at positions 5, 6 and 7 of the seed region of the antisense strand is represented by formula (V) or formula (VI).

11. The siRNA according to claim 7 or 8, **characterized in that** in the 5' to 3' direction, a nucleotide at position 5 of the seed region of the antisense strand is represented by formula (V) or formula (VI).

12. The siRNA according to claim 7 or 8, **characterized in that** in the 5' to 3' direction, a nucleotide at position 6 of the seed region of the antisense strand is represented by formula (V) or formula (VI).

13. The siRNA according to claim 7 or 8, **characterized in that** in the 5' to 3' direction, a nucleotide at position 7 of the seed region of the antisense strand is represented by formula (V) or formula (VI).

14. The siRNA according to claim 7 or 8, **characterized in that** in the 5' to 3' direction, a nucleotide at position 5 of the seed region of the antisense strand is represented by formula (V) or formula (VI), and nucleotides at positions 2, 6, 14 and 16 of the antisense strand are each independently a 2'-fluoro-modified nucleotide.

15. The siRNA according to claim 7 or 8, **characterized in that** in the 5' to 3' direction, a nucleotide at position 6 of the seed region of the antisense strand is represented by formula (V) or formula (VI), and nucleotides at positions 2, 14 and 16 of the antisense strand are each independently a 2'-fluoro-modified nucleotide.

16. The siRNA according to claim 7 or 8, **characterized in that** in the 5' to 3' direction, a nucleotide at position 7 of the seed region of the antisense strand is represented by formula (V) or formula (VI), and nucleotides at positions 2, 6, 14 and 16 of the antisense strand are each independently a 2'-fluoro-modified nucleotide.

17. A pharmaceutical composition, comprising:
the siRNA according to any one of claims 7-16; and
a pharmaceutically acceptable carrier.

18. An siRNA conjugate, comprising:
a conjugate molecule; and
the siRNA according to any one of claims 7-16 conjugated to the conjugate molecule.

19. The siRNA according to any one of claims 7-16, the pharmaceutical composition according to claim 17, and/or the siRNA conjugate according to claim 18 for use in the preparation of a drug.
